# EUROPEAN PATENT SPECIFICATION

(11) **EP 1 379 714 B1**
(45) Date of publication and mention of the grant of the patent: **31.10.2007**
(21) Application number: 02706941.8
(22) Date of filing: 27.03.2002
(51) Int. Cl.: D01F 4/02, A61L 27/22

(54) **PROCESS FOR FORMING SILK-BASED FIBRE**
VERFAHREN ZUR HERSTELLUNG VON FASERN AUF DER BASIS VON SEIDE
PROCEDE POUR LA FABRICATION DE FIBRES A BASE DE SOIE

(30) Priority: 02.04.2001 GB 0108181
(43) Date of publication of application: 14.01.2004
(73) Proprietor: Xiros Plc, Leeds LS2 9HD (GB)
(72) Inventor: CRIGHTON, John Stephen, Leeds LS2 9HD (GB)
(74) Representative: Atkinson, Jonathan David Mark
(86) International application number: PCT/GB2002/001188
(87) International publication number: WO 2002/081793

(56) References cited:
- EP-A- 0 352 330
- WO-A-92/09695
- WO-A-93/15244
- GB-A- 675 299
- US-A- 1 990 588
- US-A- 2 471 356

## Description

The present invention relates to a process for forming bioabsorbable material comprising a polymer made by the reforming of silk continuous filaments. The resultant material, which is reformed as continuous filaments from natural silk or silk waste, or from other silk-like materials, may be used in the development of tissue engineering ligaments and tendons for clinical use. The resultant material may thus be used as scaffolds for tissue engineered products and for woven and non-woven bio absorbable implants such as hernia patches, sutures, ligament retaining devices and vascular grafts. The reformed silk polymer also finds use as a vehicle for the controlled release of drugs on account of the slow degradation of the material matrix.

"Silk" is a broad description for the extruded continuous double filaments produced by a variety of animals. These filaments are protein based. The latter reflect the bulk of natural silks, with some (from the gooseberry sawfly *Nemetus ribesii*) being of collagen, but the bulk are composed of fibroin.

Silks have been used as a textile raw material since the Huang dynasty through the sericulture of the silkworm, *Bombyx mori.* It is the filamental product from the protective cocoons generated by the silkworm during its metamorphosis to the silk fly/moth which is recognised as commercial silk. The "bave" continuous double filament, coated with a ca. 30% gummy sericin protein is "spun" by the worm to yield a filament reinforced cocoon around itself as protection during its metamorphosis to the adult moth. For textile end-use this metamorphosis is halted by suffocating the moth before its potential escape from its cocoon, and this preserves filament continuity. After softening the outer layers of the cocoon in warm water, two sets of the fibroin double filaments from several (ca. 7) cocoons are concurrently twisted/wound in the unreeling operation. The ultimate "continuous" filament yarns are approx. 2 km in length with diameters of 15-25 microns (and 7 filaments).

The rest (the silk brushed from the cocoon outer surfaces to create/generate the basic continuous regular double filament, the unusable inner last elements, and broken/damaged cocoon parts) are unsuitable for use in filament yarns and are known as "waste silk". This material, when cut to short lengths, was until recently, the basis for production of "spun silk" textile yarns of woollen character.

On account of its particular mechanical properties and biocompatibility, silk filament fibres have been used for medical sutures for many years. Silk fibre is composed of fibroin protein in which the molecules are significantly orientated. Natural silk as a suture does lose strength in the body with time, however, due to enzymatic attack at susceptible and accessible sites within the amorphous component of the fibre structure.

US-A-1990588 discloses silk fibres produced from filtered silk fibroin solution.

US Patent No 4818291 describes a novel adhesive which contains a mixture of silk fibroin and human fibrinogen which is suitable for use in surgery. This invention describes mixtures containing between 5 and 90% of silk fibroin by weight, and their use as an adhesive which provides good adhesion to surfaces for medical applications. Silk fibroin is dissolved in a saturated aqueous salt solution which is then removed by dialysis. The residual fibroin suspension is then mixed with fibrinogen to produce the adhesive. The disadvantage of this process is that during the formation of the suspensions, protein degradation occurs. The resulting adhesives often have some physical properties which are poorer than those required.

US Patent No 5252285 describes a process for spinning silk fibres which also involves dissolving silk fibroin in an aqueous salt solution. The salt is removed from the solution followed by removal of the water by casting or evaporation to form an essentially amorphous silk film. This material is then dissolved in hexafluoroisopropanol to form a "solution" which is then spun and drawn to produce reformed silk fibres. This solvent, when used as described, reduces the problems of fibroin degradation. Increasingly, environmental concerns mean that there is a trend away from organic solvents such as hexafluoroisopropanol because of the cost and environmental consequences associated with their use.

Currently available bioabsorbable materials are not suitable for devices which need tensile strength and stiffness comparable to those of non-absorbable polymers such as polyester. After implantation into the human body these materials do not retain their mechanical utility for longer than 3-6 months.

This limited life is a serious disadvantage in scaffolds for load bearing structures such as ligaments and tendons which must retain a significant portion of their strength for 1-2 years whilst natural or tissue engineered cell ingrowth occurs during the healing process in order to lead to a fully repaired structure.

There is a need therefore for materials which provide controlled bio-absorbability over an extended period of time, but which also retain a sufficient degree of strength and mechanical integrity for at least a year. At the same time the material must be compatible with physiological media and be tolerated by the body. To avoid the need for surgical removal after use, the material should eventually degrade.

One object of the present invention is to achieve a combination of slow absorption by the body whilst sustaining sufficient mechanical strength to enable the material to act as a load bearing bioabsorbable fibre. This material would thus find use in medical applications, such as tissue engineered ligaments and tendons, bioabsorbable fixation systems and absorbable load bearing sutures.

Another object of the present invention is to transform waste silk material into useful medical materials. It is a further object of the invention to produce fibres derived from silk or silk wastes which have a spectrum of both mechanical properties and lifetimes within a physiological environment. It is also an aim of the present invention to produce materials of relatively low cost.

Another object of the present invention is to provide a method of spinning silk-like proteins produced from sources other than the natural silkworm, for example the process of the invention can be applied to silk obtained from genetically modified organisms such as genetically modified silkworms. The process is also applicable to synthesised fibroins such as those produced by standard genetic engineering techniques. Sources of silk protein can include genetically modified plants, such as tomato or tobacco plants, animals such as genetically modified lactating sheep, goats or bovines, or microorganisms such as genetically modified yeasts or bacteria.

A further object of the invention is to provide an amorphous support matrix to provide controlled release of pharmaceuticals. The reformed silk can be employed as a carrier in differing physical forms, which include those products of spinning/extrusion.

The present invention achieves the above aims and avoids many of the problems associated with prior art process. In particular, the present invention is able to vary the mechanical properties and to increase the lifetime of the fibre in physiological media by carefully controlling the process parameters when producing the fibre.

According to one aspect of the present invention, there is provided a process for forming a silk fibre from the product of one or more arthropod species, the process comprising steps of:
(1) providing a solvent which is a mixture of dichloroacetic acid and chloroform and/or dichloromethane,
(2) filtering the silk fibroin solution,
(3) spinning the filtered solution into a coagulation bath to produce an extruded fibre, and
(4) drawing the extruded fibre.

Preferably the coagulation bath contains alcohol.

The arthropod species include wild or cultivated silk worms (such as *Bombyx,* tussah or anaphe silk) and spiders, whether genetically modified or not; and the product may be traditionally produced, directly harvested from the silk glands of the arthropod, or obtained in any other way.

In an embodiment, the silk is of insect origin such as from spider or moth. More preferably, the silk is of moth origin, including *Bombyx,* tussah or anaphe silk. Silks from differing geographical locations and from differing silk moth and spider species can have different chemical compositions, and molecular weights; all may be used in the process of the present invention, yielding products with alternative characteristics.

In another embodiment, the silk originates from a genetically modified organism.

In an embodiment, the process comprises the following steps:
(1) optionally de-gumming the silk material and purification,
(2) cutting the silk into sub-millimetre lengths,
(3) dissolving the cut silk in a suitable solvent and precipitating the fibroin in an amorphous form,
(4) re-dissolving the cut silk in a solvent comprising a mixture of dichloroacetic acid and chloroform and/or dichloromethane to form a homogenous silk fibroin solution,
(5) filtering and de-aerating the silk fibroin solution,
(6) dry jet wet spinning or wet spinning the filtered solution into an alcohol coagulation bath to produce a coherent extruded fibre,
(7) removal of chlorinated solvents, and
(8) drawing the extruded fibre.

The drawn fibre is then washed and dried. Amorphous fibroin could be employed in the above process as the starting material and this would avoid the need to use steps 1-3 above.

The silk used as the initial material for dissolution may be any type of silk including: natural silk, waste silk or a silk pre-prepared as an amorphous film. We found that the rate of dissolution of silk prepared as an amorphous film is far more rapid than of silk fibroin fibres, thus reducing that time during which degradation can occur. This technique minimises degradation and thus achieves products with higher performance.

The silk used in the present invention is cut using a cutting mill or the like and this represents an important aspect of the present invention. Another aspect of the present invention thus provides cutting raw silk into sub-millimetre lengths using a cutting mill or the like. We have found that the use of a hammer mill as is conventionally used is deleterious to the final silk fibre because it damages the molecular integrity of the fibroin.

### The Process Overview

In an embodiment, the process thus comprises the steps of:-
(1) optionally de-gumming the silk to remove sericin;
(2) an optional purification step;
(3) converting the fibroin to an appropriate physical and/or morphological form;
(4) selecting a solvent system and dissolving the silk, preferably with minimum degradation, to form a homogenous silk fibroin solution ;
(5) filtering the silk fibroin solution,
(6) de-aeration,
(7) dry jet wet spinning or wet spinning the filtered solution into a selected liquid coagulation bath to produce a coherent extruded fibre;
(8) optional ageing;
(9) drawing the extruded fibre;
(10) final two-stage washing
(11) drying.

Some degradation of silk takes place during the preliminary processing of the silk from its natural raw state through to the viscous liquid for extrusion/spinning. Oxidation is the main cause of silk damage, and the use of an inert atmosphere, e.g. nitrogen, during the production stages reduces the extent of this effect.

The process of the present invention involves forming a silk fibroin solution by first cleaning the raw material, removing the sericin, cutting to ultra-short (i.e. sub-millimetre) lengths, and then dissolving it in a suitable solvent system to separate the histological polymer mass into constituent molecular parts. The solution is filtered and de-gassed, before being spun and drawn. Finally, the fibre is purified and dried. The reformed silk polymer of the present invention is thus made by dismantling the natural silk morphology to its component molecules, and their re-assembly via spinning/drawing operations into filaments which are slowly absorbable by the body. The rate of degradation of the fibre and hence the rate of loss of the tensile strength can be lower than that of natural silk.

In a further embodiment, the silk material is optionally subjected to modification by either introduction of inter-molecular cross-links, grafting to fibroin, or chemical modification of amino-acid residues within accessible volumes at or close to those sites where degradation in use is suspected to occur. These modifications may be achieved before dissolution of the initial silk material, or on the reformed fibre, i.e. before or after the process is effected.

Chemical modification (before or after spinning) of the reformed silk material produced by the process of the invention can be effected to alter the mechanical and biological properties of the fibre for particular medical or other purposes. Thus, for example, the strength or rate of biodegradation may be altered. This modification may be effected using one or more of the following:
(1) cross-linking with epoxides, acid anhydride acylation, aldehydes, or ethylene glycol diglycidyl ether;
(2) grafting to the protein using poly hydroxy ethyl methacrylate;
(3) gas plasma treatment with various gases with subsequent reaction with modifying agents; and
(4) prior to spinning or to drawing the fibre, increasing the amorphous volume by modifying the silk amino-acid residues.

The silk fibre can be used in medicine. A reformed silk fibre according to the invention having enhanced physical and biological properties is suitable for a number of applications in which a strong textile material is needed and is particularly suitable for any of the following applications:
(1) as a bioabsorbable surgically implantable material with tailored length of survival in the human body; or
(2) as a bioabsorbable surgically implantable material with tailored strength or modulus; or
(3) as a bioabsorbable surgically implantable material suitable for drug release applications; or
(4) as a textile material including a degree of biodegradability.

### Process steps

The production of the reformed filaments may be performed either step-wise in discrete stages or as a continuous operation.

### Extrusion

The concentration of silk dissolved in the solvent system and the composition of that solvent also influence the fibre product, and both can be varied to optimise the strength and/or lifetime of the fibre in physiological media for specific end-uses.

The silk fibroin solution for the basic initial extrusion step of the fibre production preferably should be prepared at a maximum temperature of not more than 40 °C, and more preferably not more than ambient, and preferably stored/used at as low a temperature as it is possible. Rates of degradation accelerate at temperatures approaching and above 50 °C.

De-aeration and filtration are essential prior to spinning. The former is achieved by centrifugation.

Maintenance of those times within the coagulation bath for the nascent filament and the temperature of this bath are important. We found that ambient temperature and lower temperatures lead to stronger fibres, but will require longer residence times. Preferably, the bath temperature is not more than 50 °C.

The air gap used in the "dry jet" wet spinning system was either 1.0cm or 2.0cm. Other gap lengths have been examined and the use of a larger air gap would enable the utilisation of a larger jet stretch environment.

The extrusion speed may vary within conventional parameters and we found a speed of 14.5 metres per min to be particularly suitable.

In another embodiment, the coagulation bath temperature and composition may be independently varied to influence the properties of the final fibre. The immersion time in the coagulation bath may influence the properties of the ultimate fibre.

### Drawing

In a further embodiment, the draw ratio may be chosen to influence the strength and other properties of the resulting reformed fibre. A higher draw ratio leads to a stronger fibre. The temperature of the draw bath for the as-spun coagulated filaments is important. If the draw temperature is raised to or above 170 °C then a more orientated filament is produced, which is stronger and less susceptible to degradation. However, a lower temperature minimises degradation during the drawing process itself. The optimum temperature will depend on the nature of the raw material, however, and the nature of the draw bath. The length of the bath may be varied in addition to or in place of the drawing speed to tailor the properties of the fibre.

### Chemical modifications

Modifications to provide reformed silks with different stabilities include either the use of dicarboxylic acid anhydrides to achieve cross-linking, or modifying the fibroin amino-acid residues through polymer grafting. These treatments can be applied either prior to dissolution of the purified silk or to the ultimate final drawn reformed silk filaments.

Plasma discharge finishing treatments using appropriate atmospheres provide a useful tool to create particular chemical functional groups. This facilitates specific chemical modification of the silk filaments surface such that a longer lifetime within the body can be achieved without loss of mechanical performance.

It is also possible to create specific synthesised polyaminoacids, either as homopolymers or as copolymers, as synthetic silk analogues. In this way more crystalline and therefore stronger and more stable products can be created. Also such chemical compositions avoid the presence of those amino acid residue sites at which enzymes and bio-degradation are most likely to arise and thus enjoy increased stability *in vivo.*

Biological and chemical activity is effectively constrained to the 30% less ordered volumes of the fibre where the more bulky polar and non-polar residues are inherently located. It is envisaged that the use of other chemical "treatments" could either "block" those fibroin sites at which chemical/enzyme degradation is most susceptible and/or enable chain cross-linking to be achieved. Post-production cross linking would impede access. The latter certainly would increase the bio-stability of the products. The rate of biodegradation can also be reduced by increasing the fibre diameter (decreasing specific surface area). Conversely, increasing the specific surface area (finer individual filaments) results in a more rapid degradation. One application of this is in tailoring the rate of delivery of an incorporated drug.

### Finishing

In another aspect of the present invention, reformed silk fibre can be obtained, having a longer lifetime in the body than that of a natural silk fibre under the same physiological conditions.

As a finishing treatment the silk can be coated with a very thin (one or two molecules only thick) polymer film, created *in situ* using photopolymerisation. This provides a barrier to base silk degradation and concurrently can be constructed from such polymers as to form potential sites for bonding wound healing "accelerants", or directly as a bound polymer with these accelerant features or characteristics. Such sites can also be generated/formed through plasma treatment.

Several areas of the process of the present invention are innovative. In particular:
1. The cutting of the silk fibre into ultra-short lengths facilitates dissolution with reduced degradation. Alternatively, the dissolution of amorphous silk film created by the use of rotary evaporation.
2. The choice of solvent for dissolution of the initial silk material is essential to the success of the process. More particularly, the choice of an organic solvent system ensures the quality of the fibre product and limits degradation as compared with that observed with aqueous salt solutions.
3. The choice of the processing parameters, such as the spinning parameters, can influence the properties of the fibre;
4. By varying the diameter of the reformed fibre, the rate of degradation *in vivo,* as well as the fibre's mechanical properties, can be altered to suit specific applications;
5. The use of chemical modification of silk fibroin in the form of cross-linking (to reduce the rate of bio-absorption) yields innovative benefits in fibre performance, which include enhanced tensile strength and an extended lifetime in physiological media;
6. Alternatively, using fibroin after a limited but controlled degradation, or enhancing the amorphous volume (by either introducing bulky amino-acid residues, or the use of particular spinning conditions) compared with standard silk, results in materials which are more rapidly degraded and absorbed for other applications;
7. Silk materials with a spectrum of *in vivo* degradation rates provide for its use as matrix carriers for controlled drug release;
8. Furthermore, the polymer of the present invention may be used in non-medical applications where a biodegradable polymer is required.

### Examples

### Example 1

Silk fibroin was dissolved in an organic solvent to give a silk fibroin solution. 65:35 v:v proportions of dichloroacetic acid with either methylene chloride or chloroform were used as solvents. The former system was used for "dry jet wet spinning" whilst the latter was used for 100% wet spinning. Comparatively, the former has yielded the stronger and more bio-stable end product. for both spinning systems. A 12% w/vol silk solution was used.

The room temperature coagulation bath was of either methanol or ethanol. Ethanol is preferred over methanol as there was reduced sticking together of the individual extruded and solidifying filaments.

The draw ratio levels were also investigated and a draw ratio of 4.5 to 1 was found to be suitable for the dry jet wet spun coagulated filaments.

The "jet" ratio (the ratio of initial filament collection rate to the actual extrusion rate) is another parameter which influences the nature of the product. In one variation the initial filaments are taken as coagulated filaments and washed in a separate operation, in running water for 24 hours before effecting the final drawing phase for the formation of the ultimate fibres. In another case, the fibres were extruded and drawn in a single step. It is advantageous to use a single integral continuous operation involving all the above components.

### Example 2

*Bombyx mori* "silk waste" was obtained from commercial Chinese silk textile manufacturers as a filamental elements, or as yarn waste. It was necessary to remove not only the 20% plus sericin gum present but also any stainings and other adventitious impurities/foreign mater. Degumming was achieved in most of this work by treatment with a 0.4% soap bath at 100 °C for 2 hours, followed by sequential washing with a sodium carbonate solution, boiling water, and finally with distilled water at 20 °C.

Alternatively, a proteolytic enzyme treatment was employed to achieve the same objective (Table 1).

**Table1 : Enzyme based degumming procedure employed with in-gum silk**

| Stage | Operation | Conditions |
|---|---|---|
| 1 | 0.8% papain in sodium bisulphite (0.8g/l) and hydrogen phosphate (0.4g/l) | Fibre/liquor ratio = 1:20, 60 mins at 70 °C |
| 2 | Wash with 1g/l sodium carbonate | 100 °C, 30 mins |
| 3 | Plentiful water wash | ambient temp, 24hr |
| 4 | a repeated distilled water wash | 70 °C |
| 5 | a triplicate distilled water wash | ambient temp |

The reformation of fibroin continuous filament from "waste silks" has been described in the prior art. One possible difference in procedure in the present invention resides in potentially being able to dispense with the need for a preliminary dialysis of the fibroin solution prior to spinning.

Dichloroacetic acid (DCA) is a solvent for poly N-carboxylic anhydrides. Trials with solvents based upon DCA (65% DCA+35% Dichloromethane and 65% DCA+35% Chloroform) resulted not only in facile solution of the fibroin but, their use respectively for dry jet wet or wet spinning into ethanol, and the formation of very promising filaments without the potential need to include a pre-dialysis. DCA based systems were the systems of choice for all subsequent work. The details of the solvent spinning procedures are shown in Table 2.

Approximately 10% w/v fibroin was dissolved in the chosen solvent and held at 15 °C for several hours, with stirring at 200-500 rpm to ensure complete solution and homogeneity.

Each solution was passed through an assembly of 3 stainless steel fine filter screens using positive nitrogen pressure, followed by a 10 min. centrifugation to degas. This avoids disruption during the spinning process. A further filtration was performed within the spinneret. A spinneret with 5 holes of 150 micron diameter was employed. Either wet spinning or dry jet wet spinning was practised into a 1.2 metre long ethanol coagulation bath.

**Table 2: Fibroin Spinning Systems and Procedures**

| A Wet Spinning (S1) | B Dry Jet Wet Spinning (S2) |
|---|---|
| 65% dichloroacetic acid | 65% dichloroacetic acid |
| 35% chloroform | 35% dichloromethane |
| 12%w/v fibroin conc. | 11%w/v fibroin conc. |
| 17.91 m./min.extrusion.speed | 13.875 m/min. extrusion. speed - air gap 1cm. |
| 3.45:1 draw ratio | 4.5:1 draw ratio |
| 6.0 tex product | 3.85 tex product |

In these examples, "in line" spinning/drawing was not practised. The rope of wet filaments from the ethanol coagulating bath were held loose for a period of time, for example 24 hours, before being drawn, on a separate stretching unit (through an 80 cm. long ethanol bath). Finally the drawn fibres were wound onto a stainless steel bobbin. Draw ratios of from 3:1 to 4:5 were employed.

Final purification was effected by soaking the final bobbins of drawn fibroin in ethanol for 10-12 hours. Then, to ensure the complete removal of all solvent and coagulant, the fibres were subjected to a through washing in flowing tap water. A final wash in distilled water was followed by an unforced drying at ambient temperatures.

To observe the mechanical properties, the final filaments were tested using a 20 mm gauge length of the filament (conditioned at 65% RH, 20°C.). Tests were effected on an Instron 1122 with a 50 mm/min. crosshead speed (250% rate of extension). Two contrasting examples of filament properties resulting from the alternative spinning conditions are illustrated in Table 3.

**Table 3: Reformed Fibroin Product Properties [c.f. "Standard" fibroin: 38 cN/tex strength, 23.4% extension. and "Waste silk": 26.3 cN/tex strength, 9.5% extension.]**

| A: Wet Spun (S1) | B: Dry-Jet Wet Spun (S2) |
|---|---|
| 6.0 tex product | 3.85 tex product |
| 20.8 cN/tex strength | 30.6 cN/tex strength |
| 14.8% extension at break | 18.2% extension at break |
| | Birefringence (Δn) x 10³ = 53.1 |

X-ray diffraction data from both the wet spun (S1), and the dry-jet wet spun (S2) samples show the strong presence of β pleated sheet order but with a reduced orientation compared with natural silk. With draw ratios of at least 4:1 the P conformation was significant.

Comparative observations of fibroins by vacuum derivative thermogravimetry (DTG) reflect the presence of oxidative degradation in the examined filaments. DTG curves of reformed fibroin samples from both spinning procedures indicate that some oxidative degradation has occurred.

Modification of the reformed filament fibroin was investigated. In marked contrast to natural silks the reformed filaments show markedly enhanced rates of bio-degradation, a consequence of the reduced levels of total order and easier access to those fibroin chain sites at which enzyme action is believed to arise. Modifications therefore were designed to provide in a controlled manner either enhanced or reduced levels of stability, allied to the meeting of specific mechanical property demands.

Two modification routes were used with the reformed silk filaments:
(a) **chemical modification** to block those amino acid residues perceived as particularly susceptible to enzymatic degradation and also potentially alter the mechanical performance, while "graft" polymerisation at or close to the bioactive sites, or as a thin coating, provide a purely physical barrier which can retard access by bio-active agents; and
(b) **Cross-linking** between fibroin molecular chains can directly enhance mechanical properties and partially block access to sites susceptible to sites of potential degradation.

### Cross-linking

### (i) Epoxides

Esterifications with epoxides principally target action at HIS, LYS, ARG, and TYR. Two epoxides were used: - 1.2 epoxy 3 phenoxy propane, and ethylene glycol diglycidyl ether (EGDGE).

### (ii) Acylation with acid anhydrides

Glutaric and succinic anhydrides were utilised. In the use of the dibasic anhydrides crosslink formation across basic sites is a possibility. Anhydride treatment also reduces photo-yellowing. Similar possibilities exist with itaconic anhydride.

### (iii) Aldehydes

Fibroin was modified by treatment with glutaraldehyde, with the potential to alter both the bio-stability and the mechanical properties.

### (iv) Bi-functional difluorochloropyrimidine derived cross-linking agent

Such agents are able to react with sensitive sites within the fibroin, and introduce cross-links which reduce accessibility.

### Polymer "Grafting"

A thin polymer film (a few molecules thick) can be created on the filament surface, which may be crosslinked to the surface with or without plasma treatment, or using ultra-violet light to crosslink the film without chemical link to the silk.

"Graftings" with poly hydroxy ethyl methacrylate (HEMA) were effected. The experimental details are reported within Table 4.

**Table 4: Fibroin modifications of reformed silk filaments**

| Sample | Treatment Agent | Initiator/ Catalyst | Conditions | Function |
|---|---|---|---|---|
| E1 | HEMA | Ammonium persulphate | Silk:Liquor= 1:50 (g : ml) | Grafting Modification |
| | | | 25 °C, 4.5 h | |
| E4 | EGDGE | 0.1 M NaOH | S:L = 1:100 (g : ml) | Modification /crosslinking |
| | (ethylene glycol diglycidyl ether) | | (aqueous isopropanol, 360 ml/l) 25 °C, 24 h | |
| E6 | succinic anhydride | | S:L = 1:100 (dmf*) (g : ml), 65 °C, 3.5 h (using or with 10% w/v anhydride). Also times of 45 m to 3 h at 65 °C. | Modification /crosslinking |
| E7 | glutaric anhydride 65 °C, | | S:L = 1:100 (dmf*) (g : ml), 65°C 3.5 hr (using or with 3.5 hr (using or with 10% w/v anhydride). Also times of 45 m to 2 h at 65 °C. | Modification /crosslinking |
| E8 | Glutaraldehyde | | S:L = 1:20 (g : ml) with pH 7.1 buffer (10% w/v aldehyde); 50 °C 1 h | Modification /crosslinking |

| | | | | |
|---|---|---|---|---|
| * dmf - form dimethyl amide | | | | |

Mechanical properties of the reformed fibroin filaments were measured on 2 cm lengths with a 250% crosshead speed. Some comparative data only are shown in Table 5 to identify the relative changes in strength and elongation at break which arise following each individual treatment; no account has been taken of the differing extents of "treatments" or of modification achieved.

Changes consequent on modification are complex, with possible chemical, morphological, and histological elements involved.

**Table 5: Fibroin Filaments: mechanical property changes following attempted modifications**

| Sample | % Strength Change | % Change in Elongation |
|---|---|---|
| Untreated reformed silk | 0.0 | 0.0 |
| Treatment E1 | -6.9 | +31.1 |
| Treatment E4 | -16.8 | +14.0 |
| Treatment E6 | +5.5 | +34.7 |
| Treatment E7 | -0.5 5 | +19.2 |

### Bio-degradation

Realistic studies in a simulated physiological environment at 37 °C. (body temperature) are required to test the attainment of the objectives of the invention. It was considered undesirable to use accelerated test conditions e.g. >37 °C. If, as is likely, more than one degradation process occurs, then temperatures removed from the optimum 37 °C. will generate distorted contributions to the behaviour.

The choice of specific enzyme/enzymes is dependent upon the need to replicate those particular environments which prevail at that location where the performance of the constructed substrate is targeted for use - e.g. leucine amino-peptidase and esterase in connective tissue, α -amylase in dental environments, and those to be found at sites applicable to drug delivery.

However, simple outline *in vitro* biodegradation tests were done with the individual proteolytic enzymes papain and α- chymotrypsin to identify the influence of modification on the rates of biodegradation.

Those observations made to monitor biodegradations in *in vitro* tests are changes in mechanical properties, mass loss and molecular composition. Mechanical properties and mass loss were here used as biodegradation monitors.

Proteolytic enzymes were here used at their optimum pH and at 37 °C, but at concentrations were orders in excess of those which would prevail in more definitive trials. To maintain the enzyme activity and concurrently avoid any consequential secondary action via the biodegradation products, such environments were replaced every 24 hours over a test period limited to 10 days.

α-Chymotrypsin (pH 7.8) actively breaks peptide bonds adjacent to PHE, TYR, LEU and TRP. In comparison, papain (pH 5-7.5) breaks peptide bonds adjacent to HIS and LYS. The degradation conditions and typical observations of mass losses and falls in strength with this enzyme environment are shown in Table 6. Enzyme activities on the modified silks are compared with those after exposure to just buffer alone. After the 10 day incubation the substrates were thoroughly washed, dried and then weighed to assess mass loss. Conditioned samples were mechanically tested with 20mm gauge lengths using the Instron 1122 as described earlier.

**Table 6: Bio-degradation of modified fibroin filaments [Conditions: 37 °C, papain / α-chymotrypsin in 20ml 10 day test; 50mg of sample with enzyme bath replenished every 24 hours]**

| Sample Filaments | Enzyme Bath | Mass Loss (%) | Strength Loss (%) |
|---|---|---|---|
| Unmodified "reformed" silk | papain | 7.25 | 14.3 |
| | α-chymotrypsin | 6.17 | 18.2 |
| Succinic anhydride treated reformed silk (E6) | papain | 3.81 | 9.76 |
| | α-chymotrypsin | 1.05 | 5.99 |
| Glutaric anhydride treated reformed silk (E7) | papain | 6.80 | 3.39 |
| | α-chymotrypsin | 0.95 | 2.01 |
| Glutaraldehyde treated reformed silk (E8) | papain | 2.83 | 3.47 |
| | α-chymotrypsin | 2.16 | 1.04 |
| EGDGE treated reformed silk (E9) | papain | 2.93 | 5.08 |
| | α-chymotrypsin | 0.00 | 2.65 |
| HEMA treated reformed silk (E1) | papain | 3.19 | 3.95 |
| | α-chymotrypsin | 0.37 | 2.79 |

### SUMMARY

The use of the positively biocompatible silk fibroin as a suture material has previously been impeded by the known biostability of the natural filaments. "Waste silk" is now readily available at a relatively low cost, and the present invention succeeds in transforming either these variable length and diameter materials or commercial filament silk into reformed continuous filament products with increased bio-stabilities and enhanced mechanical performance.

Either chemical modification of, or "grafting" to, the reformed fibroin has been shown to enhance bio-stability without marked loss of mechanical performance. The products can be controlled via the basic production conditions and subsequent treatments to provide products with a spectrum of biostabilities.

## Claims

1. A process for forming a silk fibre from the product of one or more arthropod species, the process comprising the steps of:
(1) providing a solvent which is a mixture of dichloroacetic acid and chloroform and/or dichloromethane,
(2) filtering the silk fibroin solution,
(3) spinning the filtered solution into a coagulation bath to produce an extruded fibre, and
(4) drawing the extruded fibre.

2. A process as claimed in claim 1, wherein the coagulation bath contains alcohol.

3. A process as claimed in claim 1 or 2, wherein the silk is of insect origin.

4. A process as claimed in any preceding claim, wherein the silk used as the initial material for dissolution may be one or more of natural silk, waste silk, silk fibroin taken directly from the insect before it is spun to form the cocoon, silk obtained from a genetically modified organism, or a silk pre-prepared as an amorphous film.

5. A process as claimed in any of claims 1 to 4, comprising the following steps:
(1) optionally de-gumming the silk material and purification;
(2) cutting the silk into sub-millimetre lengths;
(3) dissolving the cut silk in a suitable solvent and precipitating the fibroin in an amorphous form;
(4) re-dissolving the cut silk in a suitable solvent and precipitating the fibroin in an amorphous form;
(5) filtering and de-aerating the silk fibroin solution;
(6) dry jet wet spinning or wet spinning the filtered solution into an alcohol coagulation bath to produce a coherent extruded fibre;
(7) removal of chlorinated solvents; and
(8) drawing the extruded fibre.

6. A process as claimed in any of claims 1 to 4, comprising the following steps:
(1) optionally de-gumming the silk to remove sericin;
(2) an optional purification step;
(3) converting the fibroin to an appropriate physical and/or morphological form;
(4) selecting a solvent system and dissolving the silk, preferably with minimum degradation, to form a homogenous silk fibroin solution;
(5) filtering the silk fibroin solution;
(6) de-aeration;
(7) dry jet wet spinning or wet spinning the filtered solution into a selected liquid coagulation bath to produce a coherent extruded fibre.

7. A process as claimed in any preceding claim, wherein the silk is chemically modified before or after the process is effected.

8. A process as claimed in claim 7, wherein the chemical modification is one or more of:
(1) cross-linking with epoxides, acid anhydride acylation, aldehydes, or ethylene glycol diglycidyl ether;
(2) grafting to the protein using poly hydroxy ethyl methacrylate;
(3) gas plasma treatment with various gases with subsequent reaction with modfying agents; and
(4) prior to spinning or to drawing the fibre, increasing the amorphous volume by modifying the silk amino-acid residues.

## Patentansprüche

1. Verfahren zur Formung einer Seidenfaser aus dem Produkt von einer oder mehreren Arthropodenarten, umfassend die Stufen:
(1) Bereitstellen eines Lösungsmittels, das ein Gemisch aus Dichloressigsäure und Chloroform und/oder Dichlormethan ist,
(2) Filtrieren der Seidenfibroinlösung,
(3) Spinnen der filtrierten Lösung in ein Koagulationsbad, um eine extrudierte Faser zu erzeugen und
(4) Verstrecken der extrudierten Faser.

2. Verfahren, wie in Anspruch 1 beansprucht, wobei das Koagulationsbad Alkohol enthält.

3. Verfahren, wie in Anspruch 1 oder 2 beansprucht, wobei die Seide von Insekten stammt.

4. Verfahren, wie in einem der vorstehenden Ansprüche beansprucht, wobei die Seide, verwendet als das Ausgangsmaterial zur Lösung, eine oder mehrere aus natürlicher Seide, Abfallseide, Seidenfibroin, das direkt vom Insekt genommen wird, bevor es zur Bildung des Kokons versponnen wird, Seide, die von einem genetisch modifizierten Organismus erhalten wird, oder eine Seide, die als amorpher Film vorpräpariert ist, sein kann.

5. Verfahren, wie in einem der Ansprüche 1 bis 4 beansprucht, umfassend die folgenden Stufen:
(1) gegebenenfalls Entleimen des Seidenmaterials und Reinigung;
(2) Schneiden der Seide auf Submillimeterlängen;
(3) Lösen der geschnittenen Seide in einem geeigneten Lösungsmittel und Fällen des Fibroins in amorpher Form;
(4) Wiederauflösen der geschnittenen Seide in einem geeigneten Lösungsmittel und Fällen des Fibroins in amorpher Form;
(5) Filtrieren und Entlüften der Seidenfibroinlösung;
(6) Dry-Jet-Wet-Spinnen oder Nassspinnen der filtrierten Lösung in ein Alkoholkoagulationsbad, um eine zusammenhängende extrudierte Faser zu erzeugen;
(7) Entfernung von chlorierten Lösungsmitteln und
(8) Verstrecken der extrudierten Faser.

6. Verfahren, wie in einem der Ansprüche 1 bis 4 beansprucht, umfassend die folgenden Stufen:
(1) gegebenenfalls Entleimen der Seide zur Entfernung von Sericin;
(2) eine optionale Reinigungsstufe;
(3) Umwandeln des Fibroins in eine geeignete physikalische und/oder morphologische Form;
(4) Auswählen eines Lösungsmittelsystems und Lösen der Seide, vorzugsweise bei minimaler Degradation, um eine homogene Seidenfibroinlösung zu bilden;
(5) Filtrieren der Seidenfibroinlösung;
(6) Entlüftung;
(7) Dry-Jet-Wet-Spinnen oder Nassspinnen der filtrierten Lösung in ein ausgewähltes flüssiges Koagulationsbad, um eine zusammenhängende extrudierte Faser zu erzeugen.

7. Verfahren, wie in einem der vorstehenden Ansprüche beansprucht, wobei die Seide vor oder nach Durchführung des Verfahrens chemisch modifiziert wird.

8. Verfahren, wie in Anspruch 7 beansprucht, wobei die chemische Modifikation eine oder mehrere ist von:
(1) Vernetzung mit Epoxiden, Säureanhydridacylierung, Aldehyden oder Ethylenglycoldiglycidylether;
(2) Pfropfung auf das Protein unter Verwendung von Polyhydroxyethylmethacrylat;
(3) Gasplasmabehandlung mit verschiedenen Gasen mit nachfolgender Umsetzung mit Modifizierungsmitteln; und
(4) vor dem Spinnen oder Verstrecken der Faser Erhöhen des amorphen Volumens durch Modifizieren der Seidenaminosäurereste.

## Revendications

1. Procédé permettant de former une fibre de soie à partir du produit d'une ou plusieurs espèces d'arthropodes, le procédé comprenant les étapes consistant à :
(1) apporter un solvant qui est un mélange d'acide dichloracétique et de chloroforme et/ou de dichlorométhane,
(2) filtrer la solution de fibroïne de soie,
(3) filer la solution filtrée dans un bain de coagulation pour produire une fibre extrudée, et
(4) étirer la fibre extrudée.

2. Procédé selon la revendication 1, dans lequel le bain de coagulation contient de l'alcool.

3. Procédé selon la revendication 1 ou la revendication 2, dans lequel la soie provient d'un insecte.

4. Procédé selon l'une quelconque des revendications précédentes, dans lequel la soie utilisée comme substance initiale pour la dissolution peut être une ou plusieurs soie(s) parmi la soie naturelle, la soie schappe, la fibroïne de soie directement prélevées sur l'insecte avant qu'elle ne soit filée pour former le cocon, la soie obtenue à partir d'un organisme génétiquement modifié, ou une soie préparée au préalable sous la forme d'un film amorphe.

5. Procédé selon l'une quelconque des revendications 1 à 4, comprenant les étapes suivantes :
(1) dégommage facultatif de la soie et purification ;
(2) découpage de la soie en longueurs inférieures au millimètre ;
(3) dissolution de la soie découpée dans un solvant approprié et précipitation de la fibroïne sous une forme amorphe ;
(4) re-dissolution de la soie découpée dans un solvant approprié et précipitation de la fibroïne sous une forme amorphe ;
(5) filtration et désaération de la solution de fibroïne de soie ;
(6) filage au mouillé à jet sec ou filage au mouillé de la solution filtrée dans un bain de coagulation à base d'alcool pour produire une fibre extrudée cohérente ;
(7) élimination des solvants chlorés ; et
(8) étirage de la fibre extrudée.

6. Procédé selon l'une quelconque des revendications 1 à 4, comprenant les étapes suivantes :
(1) dégommage facultatif de la soie pour éliminer la séricine ;
(2) étape facultative de purification ;
(3) transformation de la fibroïne en une forme physique et/ou morphologique appropriée ;
(4) sélection d'un système de solvants et dissolution de la soie, de préférence avec un minimum de dégradation, pour former une solution de fibroïne de soie homogène ;
(5) filtration de la solution de fibroïne de soie ;
(6) désaération ;
(7) filage au mouillé à jet sec ou filage au mouillé de la solution filtrée dans un bain de coagulation liquide choisi pour produire une fibre extrudée cohérente.

7. Procédé selon l'une quelconque des revendications précédentes, dans lequel la soie est chimiquement modifiée avant ou après que le procédé soit effectué.

8. Procédé selon la revendication 7, dans lequel la modification chimique est l'une ou plusieurs des modifications suivantes :
(1) réticulation avec des époxydes, acylation avec un anhydride d'acide, des aldéhydes, ou de l'éther diglycidylique d'éthylèneglycol ;
(2) greffage de la protéine à l'aide de polyméthacrylate d'hydroxyéthyle ;
(3) traitement au plasma avec divers gaz et réaction subséquente avec des agents modificateurs ; et
(4) avant filage ou étirage de la fibre, augmentation du volume amorphe par modification des résidus d'acides aminés de la soie.
